# EUROPEAN PATENT APPLICATION

(11) **EP 1 967 855 A1**
(43) Date of publication of application: **10.09.2008**
(21) Application number: 07103782.4
(22) Date of filing: 08.03.2007
(51) Int. Cl.: G01N 33/543

(54) **Magnetic sensor device**

(71) Applicant: Koninklijke Philips Electronics N.V., 5621 BA Eindhoven (NL)
(72) Inventor: The designation of the inventor has not yet been filed
(74) Representative: van Wermeskerken, Stephanie Christine

(57) **Abstract**

The present invention provides a magnetic sensor device (20) for determining the presence and/or amount of target moieties (32) in a sample fluid, the target moieties being labeled with magnetic or magnetizable objects (33). The magnetic sensor device (20) comprises a sensor surface (23) lying in a plane, at least one magnetic element (21) for providing a sensor signal and at least one structure (24) extending from the sensor surface (23) in a direction substantially perpendicular to the plane of the sensor surface (23), the at least one structure (24) having a height (h) and a binding surface (25) for binding the labeled target moieties (32). The height (h) of the at least one structure (24) is higher than zero and lower or equal to a distance (z) of the labeled target moieties (32) from the sensor surface (23) corresponding to a signal cross-over point (27) from positive to negative signal contribution of the labeled target moieties (32) to the sensor signal, the signal cross-over point (27) being the distance at which a homogeneous distribution of magnetic or magnetizable objects (33) between the sensor surface (23) and that distance has a signal contribution to the sensor signal equal to zero.

## Description

### FIELD OF THE INVENTION

The present invention relates to magnetic sensor devices. More particularly, the present invention relates to magnetic sensor devices having structures extending in a direction substantially perpendicular to a plane of a sensor surface, to a method for manufacturing of such magnetic sensor devices and to a method for determining the presence and/or amount of target moieties in a sample fluid, the target moieties being labeled with magnetic or magnetizable objects. The device and methods according to embodiments of the invention can be used in molecular diagnostics, biological sample analysis or chemical sample analysis.

### BACKGROUND OF THE INVENTION

Magnetic sensors based on AMR (anisotropic magneto resistance), GMR (giant magneto resistance) and TMR (tunnel magneto resistance) elements or on Hall sensors, are nowadays gaining importance. Besides the known high-speed applications such as magnetic hard disk heads and MRAM, new relatively low bandwidth applications appear in the field of molecular diagnostics (MDx), current sensing in IC's, automotive, etc.

The introduction of micro-arrays or biochips comprising such magnetic sensors is revolutionizing the analysis of biomolecules such as DNA (desoxyribonucleic acid), RNA (ribonucleic acid) and proteins. Applications are, for example, human genotyping (e.g. in hospitals or by individual doctors or nurses), bacteriological screening, biological and pharmacological research. Such magnetic biochips have promising properties for, for example, biological or chemical sample analysis, in terms of sensitivity, specificity, integration, ease of use and costs.

Biochips, also called biosensor chips, biological microchips, gene-chips or DNA chips, consist in their simplest form of a substrate on which a large number of different probe molecules are attached, on well-defined regions on the chip, to which molecules or molecule fragments that are to be analyzed can bind if they are perfectly matched. For example, a fragment of a DNA molecule binds to one unique complementary DNA (c-DNA) molecular fragment. The occurrence of a binding reaction can be detected, for example by using markers, e.g. fluorescent markers or magnetic labels, which are coupled to the molecules to be analyzed, either before or after binding of these molecules to the probe molecules. This provides the ability to analyze small amounts of a large number of different molecules or molecular fragments in parallel, in a short time.

In a biosensor an assay takes place. Assays generally involve several fluid actuation steps, i.e. steps in which materials are brought into movement. Examples of such steps are mixing (e.g. for dilution, or for the dissolution of labels or other reagents into the sample fluid, or for labeling, or for affinity binding) or the refresh of fluid near to a reaction surface in order to avoid that diffusion becomes rate-limiting for the reaction. Preferably the actuation method should be effective, reliable and cheap.

A biosensor consisting of an array of, for example 10, sensors based on the detection of e.g. superparamagnetic beads may be used to simultaneously measure the concentration of a plurality of different biological molecules (e.g. protein, DNA) in a solution (e.g. blood). This may be achieved by attaching a superparamagnetic bead to target molecules which are to be determined, magnetizing this bead with an applied magnetic field and using e.g. a Giant Magneto Resistance (GMR) sensor to detect the magnetic field of the magnetized beads.

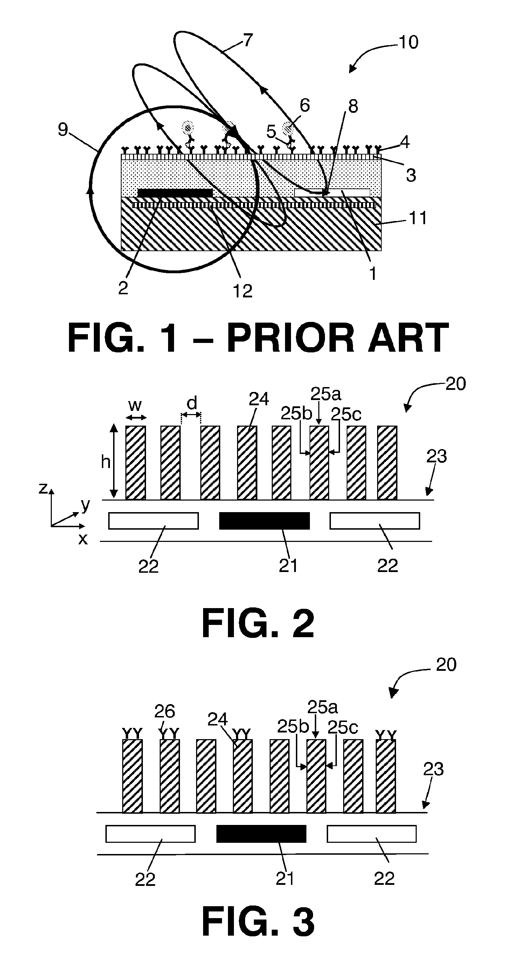

Fig. 1 illustrates the working principle of a magnetoresistive biosensor configuration with integrated magnetic field excitation as presently known in the art. The magnetoresistive biosensor 10 comprises a single GMR strip 1, e.g. with a length of about 100 µm and a width of about 3 µm. With integrated magnetic field excitation is meant that a magnetic field generating means is integrated in the magnetoresistive sensor 10. The magnetoresistive sensor 10 furthermore comprises a current wire 2 as magnetic field generating means. At a surface 3 of the magnetoresistive biosensor 10, a bioactive layer of binding sites 4 are provided to which, for example, target molecules 5 with attached thereto a magnetic bead 6, e.g. a superparamagnetic nanoparticle, can bind. A current flowing through the current wire 2 generates a magnetic field 9 which magnetizes the magnetic bead 6, e.g. superparamagnetic nanoparticle. The magnetic bead 6, e.g. magnetic nanoparticle, develops a magnetic moment m indicated by field lines 7 in Fig. 1. The magnetic moment m then generates dipolar magnetic fields, which have in-plane magnetic field components 8 at the location of the GMR strip 1. Thus, the magnetic bead 6, e.g. magnetic nanoparticle, deflects the magnetic field 9 induced by the current through the current wire 2, resulting in the magnetic field component 8 in the sensitive x-direction of the GMR strip 1, also called x-component 8 of the magnetic field H. The x-component of the magnetic field H is then sensed by the GMR strip 1 and depends on the number Nₙₚ of magnetic beads 6, e.g. superparamagnetic nanoparticles, present at the surface 3 of the magnetoresistive biosensor 10 and on the magnitude of the current in the current wire 2. The magnetoresistive biosensor 10 may be formed on a semiconductor chip, e.g. silicon chip 11, in which electronic circuitry 12 may be embedded.

The majority of biological test kits that are currently on the market make use of a so-called lateral flow assay. In this assay a porous material (generally nitrocellulose) is used as a substrate of a sensor device to achieve a capillary flow of fluid through the porous material. At a position in the porous material a detection area is defined by coating binding molecules onto the porous material. The binding of particles to target molecules immobilized at a surface of the substrate will cause a coloring of the porous strip. This coloring can be measured optically. The advantage of the use of a porous material as a substrate in a sensor device is that a relatively constant 'creep-flow' can be sustained at the position of possible binding sites. Furthermore, a large surface-to-volume ratio may be realized. This considerably improves the efficiency of binding target molecules. It is, however, known that inhomogeneity in the porous material can be a large cause of variation in the assay result. The variation in the assay result can for example be caused by local clustering of particles in the porous material, by the local absence of immobilized binding molecules in the porous material or because of the surface area-to-volume ratio is differing from position to position or from batch to batch.

WO 01/62887 provides a sensor with a high surface-to-volume ratio by providing a chip comprising a base including a non-sample surface and at least one structure comprising a pillar and a sample surface that is elevated with respect to the non-sample surface and is adapted to receive a sample from a dispenser. The height of each pillar may preferably be higher than 1 µm. For example, the height of each pillar may range from about 1 to 10 µm, or from 10 to 200 µm. Each pillar may have any suitable width including a width of less than about 0.5 mm (e.g. 100 µm or less). The device according to WO 01/62887 may be used for detecting whether analytes in a fluid bind to binding sites on the pillars of the sensor or not, and for detecting the amount of analytes bound to the surface of the sensor. Therefore, according to embodiments of WO 01/62887, fluorescent tags may be attached to the analytes. Detection of the analytes which are bound to the binding sites on the pillars may then be performed by fluorescence, fluorescence polarization, surface plasmon resonance (SPR), imaging SPR, ellipsometry or imaging ellipsometry.

However, when such structures are applied to surfaces of magnetic sensor devices for detecting analytes by labeling them to a magnetic particle and applying a magnetic field, a decrease of the sensitivity of the sensor device and an incorrect value for the detected amount of analytes derived from the sensor signal has been noticed.

### SUMMARY OF THE INVENTION

It is an object of the present invention to provide a good magnetic sensor device, a method for the manufacturing of such a magnetic sensor device and a method for detecting and/or quantifying magnetic or magnetizable objects in a sample fluid.

The above objective is accomplished by a method and device according to the present invention.

The magnetic sensor device and methods according to embodiments of the invention show good sensitivity and can be used for detecting and/or quantifying low amounts of target moieties in a sample fluid, e.g. 1 pM to 10 pM.

The magnetic sensor device and method according to embodiments of the present invention may be used in molecular diagnostics, biological sample analysis or chemical sample analysis.

In a first aspect, the invention provides a magnetic sensor device for determining the presence and/or amount of target moieties in a sample fluid, the target moieties being labeled with magnetic or magnetizable objects. The magnetic sensor device comprises:
- a sensor surface lying in a plane,
- at least one structure extending from the sensor surface in a direction substantially perpendicular to the plane of the sensor surface, the at least one structure having
- a height and a binding surface for binding labeled target moieties, and
- at least one magnetic sensor element for providing a sensor signal representative for the presence and/or amount of labeled target moieties bound to the binding surface of the at least one structure,
wherein the height of the at least one structure is higher than zero and lower than or equal to a distance of the labeled target moieties from the sensor surface corresponding to a signal cross-over point from positive to negative signal contribution of the labeled target moieties to the sensor signal, the signal cross-over point being the distance at which a homogeneous distribution of magnetic or magnetizable objects between the sensor surface and that distance has a signal contribution to the sensor signal equal to zero.

An advantage of the magnetic sensor device according to embodiments of the present invention is that it shows a high sensitivity, high sensor signal, a good surface-to-volume ratio above the sensor surface while maintaining a creep-flow over the sensor surface by capillary actuation of the fluid.

According to embodiments of the invention, the height of the at least one structure may be equal to the distance of the labeled target moieties from the sensor surface corresponding to the signal cross-over point from positive to negative signal contribution of the labeled target moieties at a distance from the sensor surface to the sensor signal.

An advantage hereof is that the contribution of the bound labeled target moieties to the sensor signal is maximal, and thus the highest possible obtainable sensor signal can be obtained.

The at least one structure may have a width which may be between 0.5 µm and 10 µm. The lower limit of the width may be limited by the technique used to form the at least one structure, such as e.g. soft lithography or hot embossing lithography.

The magnetic sensor device may comprise a plurality of structures and there may be a distance between neighboring structures. According to embodiments of the invention, the distance between neighboring structures may be between 1 µm and 70 µm. The distance between neighboring structures may preferably be such that a capillary flow of sample fluid over the sensor surface is obtained

According to embodiments of the invention, the magnetic sensor device may comprise a plurality of structures and the plurality of structures may be arranged over the sensor surface in a matrix, the matrix comprising rows and columns. The matrix may be a matrix of aligned or of staggered lines.

According to other embodiments of the present invention, the plurality of structures may be randomly arranged over the sensor surface.

The magnetic sensor device may furthermore comprise magnetic field generating means. The magnetic field generating means may, according to embodiments of the invention, be an on-chip or integrated magnetic field generating means formed by, for example, at least one current wire. According to other embodiments of the invention, the magnetic field generating means may be an off-chip magnetic field generating means, such as, for example, an external coil.

The at least one structure may have an upper surface and at least one side surface. The binding surface of the at least one structure may be formed by its upper surface and/or its at least one side surface. When the height of the at least one structure is equal to the distance of the labeled target moieties from the sensor surface corresponding to a signal cross-over point, the binding surface of the at least one structure may preferably be formed by at least one side surface because labeled target moieties bound to the upper surface of the at least one structure will have a signal contribution of zero. When the height of the at least one structure is lower than the distance of the labeled target moieties from the sensor surface corresponding to a signal cross-over point, the binding surface of the at least one structure may preferably be formed by its upper surface and at least one side surface, because in this case also labeled target moieties bound to the upper surface of the at least one structure will positively contribute to the sensor signal, in that way enhancing the sensor signal and thus enhancing the sensitivity of the magnetic sensor device.

According to embodiments of the present invention, at least one of the upper surface and the at least one side surface of the at least one structure may comprise specific binding sites.

The at least one structure may be formed of a polymer material, e.g. an SU-8 polymer, or may be formed of an inorganic material, e.g. SiO₂ or Si₃N₄.

The at least one structure may have a rectangular, a cylindrical or a conical shape in cross-section. The at least one structure may have an irregular shape in cross-section.

The magnetic or magnetizable objects may be magnetic particles.

In a second aspect of the present invention, the use of the magnetic sensor device according to embodiments of the present invention in molecular diagnostics, biological sample analysis or chemical sample analysis is provided.

In a third aspect of the present invention, a biochip comprising at least one magnetic sensor device according to embodiments of the present invention is provided.

The biochip according to embodiments of the invention may be used in molecular diagnostics, biological sample analysis or chemical sample analysis.

In a further aspect of the invention, a method for manufacturing a magnetic sensor device is provided. The method comprises:
- providing at least one structure on a sensor surface lying in a plane, the at least one structure extending from the sensor surface in a direction substantially perpendicular to the plane of the sensor surface over a height and having a binding surface for binding target moieties labeled with magnetic or magnetizable objects, and
- providing at least one magnetic sensor element for providing a sensor signal representative for the presence and/or amount of labeled target moieties bound to the binding surface of the at least one structure,
wherein providing at least one structure is performed such that the height of the at least one structure is higher than zero and lower than or equal to a distance of the labeled target moieties from the sensor surface corresponding to a signal cross-over point from positive to negative signal contribution of the labeled target moieties, the signal cross-over point being the distance at which a homogeneous distribution of magnetic or magnetizable objects between the sensor surface and that distance has a signal contribution to the sensor signal equal to zero.

According to embodiment of the present invention providing at least one structure may comprise:
- providing a layer of polymer material onto the sensor surface, and
- patterning the layer of polymer material.

According to specific embodiment, providing a layer of polymer material may be performed by providing a layer of SU-8 polymer.

Patterning the layer of polymer material may be performed by an imprinting technique such as hot-embossing or by lithographic techniques such as by photolithographic patterning.

Providing at least one structure may be performed such that the height of the at least one structure is equal to the distance of the labeled target moieties from the sensor surface corresponding to the signal cross-over point from positive to negative signal contribution of the labeled target moieties to the sensor signal.

The method may comprise providing a plurality of structures. According to embodiments of the invention, the plurality of structures may be arranged over the sensor surface in a matrix, the matrix comprising rows and columns. The matrix may be a matrix of aligned or of staggered lines. According to other embodiments, the plurality of structures may be randomly arranged over the sensor surface.

In yet a further aspect of the invention, a method is provided for determining the presence and/or amount of target moieties in a sample fluid. The method comprises:
- providing a sample fluid comprising labeled target moieties to a magnetic sensor device comprising a sensor surface lying in a plane, at least one magnetic element for providing a sensor signal, and at least one structure having a height higher than zero and lower or equal to a distance of the labeled target moieties from the sensor surface corresponding to a signal cross-over point from positive to negative signal contribution of the labeled target moieties to the sensor signal, the signal cross-over point being the distance at which a homogeneous distribution of magnetic or magnetizable objects between the sensor surface and that distance has a signal contribution to the sensor signal equal to zero,
- applying a magnetic field for magnetizing the magnetic or magnetizable objects bound to target moieties bound to the binding surface of the at least one structure,
- measuring the sensor signal by means of the magnetic element, and
- from the measured sensor signal determining the presence and/or amount of target moieties bound to the binding surface of the at least one structure.

According to embodiment of the invention, providing the sample fluid comprising labeled target moieties to the sensor device may comprise:
- first applying a sample fluid comprising target moieties to the magnetic sensor device, and
- thereafter adding magnetic or magnetizable objects to the sample fluid.

According to other embodiments of the invention, providing the sample fluid comprising labeled target moieties to the sensor device may comprise simultaneously applying a sample fluid comprising target moieties to the magnetic sensor device and adding magnetic or magnetizable objects to the sample fluid.

According to still other embodiments, providing the sample fluid comprising labeled target moieties to the sensor device may comprise:
- first adding magnetic or magnetizable objects to a sample fluid comprising target moieties, and
- thereafter applying the sample fluid to the magnetic sensor device.

A disadvantage of these latter embodiments is that it cannot be applied in case of a competitive assay.

In a further aspect of the present invention, the use of the method for determining the presence and/or amount of target moieties in a sample fluid according to embodiments of the invention in molecular diagnostics, biological sample analysis or chemical sample analysis is provided.

Particular and preferred aspects of the invention are set out in the accompanying independent and dependent claims. Features from the dependent claims may be combined with features of the independent claims and with features of other dependent claims as appropriate and not merely as explicitly set out in the claims.

The above and other characteristics, features and advantages of the present invention will become apparent from the following detailed description, taken in conjunction with the accompanying drawings, which illustrate, by way of example, the principles of the invention. This description is given for the sake of example only, without limiting the scope of the invention. The reference figures quoted below refer to the attached drawings.

### BRIEF DESCRIPTION OF THE DRAWINGS

Fig. 1 illustrates the principle of a known magnetic biosensor.
Fig. 2 is a side view of a biosensor according to an embodiment of the present invention.
Fig. 3 is a side view of a biosensor according to an embodiment of the present invention.
Fig. 4 shows signal contribution of magnetic particles as a function of their distance above a sensor surface.
Fig. 5 is a top view of a biosensor according to an embodiment of the present invention.
Fig. 6 is a top view of a biosensor according to another embodiment of the present invention.
Figs. 7 and 8 illustrate suitable shapes for structures suitable for use in a magnetic sensor device according to embodiments of the present invention.
Fig. 9 illustrates the stability of SU-8 coated surfaces over time.
Fig. 10 illustrates a biochip comprising at least one magnetic sensor device according to embodiments of the invention.

In the different Figures, the same reference signs refer to the same or analogous elements.

### DETAILED DESCRIPTION OF EMBODIMENTS

The present invention will be described with respect to particular embodiments and with reference to certain drawings but the invention is not limited thereto but only by the claims. Any reference signs in the claims shall not be construed as limiting the scope. The drawings described are only schematic and are non-limiting. In the drawings, the size of some of the elements may be exaggerated and not drawn on scale for illustrative purposes.

Where the term "comprising" is used in the present description and claims, it does not exclude other elements or steps. Where an indefinite or definite article is used when referring to a singular noun e.g. "a" or "an", "the", this includes a plural of that noun unless something else is specifically stated.

Furthermore, the terms first and second and the like in the description and in the claims, are used for distinguishing between similar elements and not necessarily for describing a sequence, either temporally, spatially, in ranking or in any other manner. It is to be understood that the terms so used are interchangeable under appropriate circumstances and that the embodiments of the invention described herein are capable of operation in other sequences than described or illustrated herein.

Moreover, the terms top, side and the like in the description and the claims are used for descriptive purposes and not necessarily for describing relative positions. It is to be understood that the terms so used are interchangeable under appropriate circumstances and that the embodiments of the invention described herein are capable of operation in other orientations than described or illustrated herein.

Reference throughout this specification to "one embodiment" or "an embodiment" means that a particular feature, structure or characteristic described in connection with the embodiment is included in at least one embodiment of the present invention. Thus, appearances of the phrases "in one embodiment" or "in an embodiment" in various places throughout this specification are not necessarily all referring to the same embodiment, but may do so. Furthermore, the particular features, structures or characteristics disclosed may be combined in any suitable manner, as would be apparent to one of ordinary skill in the art from this disclosure, in one or more embodiments.

Similarly it should be appreciated that in the description of exemplary embodiments of the invention, various features of the invention are sometimes grouped together in a single embodiment, figure, or description thereof for the purpose of streamlining the disclosure and aiding in the understanding of one or more of the various inventive aspects. This method of disclosure, however, is not to be interpreted as reflecting an intention that the claimed invention requires more features than are expressly recited in each claim. Rather, as the following claims reflect, inventive aspects lie in less than all features of a single foregoing disclosed embodiment. Thus, the claims following the detailed description are hereby expressly incorporated into this detailed description, with each claim standing on its own as a separate embodiment of this invention.

Furthermore, while some embodiments described herein include some but not other features included in other embodiments, combinations of features of different embodiments are meant to be within the scope of the invention, and form different embodiments, as would be understood by those skilled in the art. For example, in the following claims, any of the claimed embodiments can be used in any combination.

In the description provided herein, numerous specific details are set forth. However, it is understood that embodiments of the invention may be practiced without these specific details. In other instances, well-known methods, structures and techniques have not been shown in detail in order not to obscure an understanding of this description.

In a first aspect of the invention a magnetic sensor device is provided for determining the presence and/or amount of target moieties in a sample fluid, the target moieties being labeled with magnetic or magnetizable objects, such as e.g. magnetic particles, for example magnetic nanoparticles. The magnetic sensor device comprises a sensor surface lying in a plane. At least one structure is extending from the sensor surface in a direction substantially perpendicular to the plane of the sensor surface. The at least one structure has a height and a binding surface for binding target moieties, e.g. labeled target moieties. The magnetic sensor device furthermore comprises at least one magnetic sensor element for, when in use, providing a sensor signal representative of the presence and/or of the amount of target moieties, e.g. labeled target moieties bound to the binding surface of the at least one structure. It has been noted that the signal contribution to the sensor signal of labeled analytes bound to such structures may be negative if they are too far away from a magnetic element used for sensing the presence of such labeled analytes. This leads to a decrease of the sensitivity of the sensor device and to an incorrect value for the detected amount of analytes derived from the sensor signal. Therefore, according to the present invention, the height of the at least one structure is determined to be higher than zero and lower than or equal to a distance of the target moieties, e.g. labeled target moieties from the sensor surface corresponding to a signal cross-over point from positive to negative signal contribution of the target moieties, e.g. labeled target moieties to the sensor signal. The signal cross-over point is located at a distance where the signal contribution of the bound target moieties, e.g. labeled target moieties to the sensor signal is equal to zero.

According to embodiments of the invention the height of the at least one structure may thus be such that all, or substantially all, the target moieties, e.g. labeled target moieties bound to the binding surface of the at least one structure positively contribute to the sensor signal. With all, or substantially all, the target moieties, e.g. labeled target moieties positively contributing to the sensor signal is meant that the presence of the target moieties, e.g. labeled target moieties bound to the binding surface of the at least one structure does not, or not substantially, cancel the signal. Therefore, with substantially all is meant at least 95%, more preferred at least 97%, still more preferred at least 99%. Thus all, or substantially all, target moieties, e.g. labeled target moieties bound to the binding surface of the at least one structure contribute to an increase of the measured sensor signal, which is representative for the presence and/or the amount of bound target moieties, e.g. labeled target moieties. In this way, the amount of target moieties which are bound to the binding surface of the at least one structure can be correctly derived from the measured sensor signal, as all, or substantially all, bound target moieties lead to an increase of the sensor signal.

Furthermore, because of the proposed configuration, with one or more structures extending from the sensor surface in a direction substantially perpendicular to the plane of the sensor surface, variations which appear in the sensor signal of sensor devices comprising a porous material as a substrate, as described earlier, can be eliminated while still maintaining creep-flow across the sensor surface by capillary actuation of the fluid comprising the target moieties.

Moreover, the presence of the at least one structure on the sensor surface increases the total binding area of the sensor surface, as target moieties can bind not only to the surface area between the structures or to the top of the structures, but also to side surfaces of the structures. As a consequence, the surface-to-volume ratio of the sensor surface is improved.

The magnetic sensor device according to the present invention can, for example, be used in molecular diagnostics, biological sample analysis or chemical sample analysis for, for example, detecting and/or quantifying target moieties present in a sample fluid and labeled with magnetic and/or magnetizable objects. Target moieties may include molecular species, cell fragments, viruses, etc.

The present invention will further be described by means of a magnetic sensor device based on magnetoresistive elements such as GMR elements as magnetic sensor elements. However, this is not intended to be limiting for the invention in any way. Any other suitable magnetic sensor elements may also be used, e.g. magnetic sensor elements or magnetoresistive sensor elements based on AMR or TMR elements, Hall sensors, coils.

The magnetic sensor device in accordance with embodiments of the present invention may furthermore comprise a magnetic field generator. The magnetic field generator may be an external magnetic field generator. The external magnetic field generator may either be in the form of a permanent magnet that may translate between at least two different physical positions with respect to the sensor surface or in the form of an electromagnet where the driving current for the electromagnet may be time varying. Alternatively, the magnetic field generator may be an internal magnetic field generator. The magnetic field generator may, for example, comprise at least one conductor such as e.g. at least one current wire. The magnetic field generator can be used for magnetizing the magnetic or magnetizable object present in the neighborhood of the magnetic sensor device and attached to target moieties to be detected. The magnetic or magnetizable object is preferably a magnetic nanoparticle, but may also be any other suitable magnetizable object, which can be attached to target moieties. The present invention will further be described by means of the magnetic or magnetizable objects being magnetic particles, more specifically magnetic nanoparticles which may preferably have a diameter of between 50 nm and 1µm. Again, this is only for the ease of explanation and does not limit the invention in any way. The present invention also applies for a magnetic or magnetizable object being a magnetic rod, a string of magnetic particles, or a composite particle, e.g. a particle containing magnetic as well as non-magnetic material, for example optically-active material, or magnetic material inside a non-magnetic matrix. Fig. 2 shows a cross-section of a magnetic sensor device 20 according to an embodiment of the present invention. The magnetic sensor device 20 according to the example given may comprise a GMR element 21 as magnetic sensor element and a first and second current wire 22 as magnetic field generator. It has to be understood that, according to other embodiments of the present invention, the magnetic sensor device 20 may also comprise any other number of sensor elements particularly magnetic sensor elements such as GMR elements 21 and/or any other number of magnetic filed generators such as one current wire 22 or any number of current wires 22 higher than two. The present invention also includes within its scope any other configuration of sensor elements particularly magnetic sensor elements such as GMR elements 21 and magnetic field generators such as current wires 22. Moreover, the magnetic sensor device 20 may, instead of an on-chip magnetic field generating means as described above, also comprise an off-chip magnetic field generating means, such as for example an external coil.

On a surface 23 of the magnetic sensor device 20, lying in a plane, the x-y-plane as illustrated in Fig. 2, the magnetic sensor device 20 comprises at least one structure 24 or having at least one surface extending from the sensor surface 23 in a direction substantially perpendicular to the plane of the sensor surface 23, or as indicated by the coordinate system in Fig. 2 in the z-direction. In the example given in Fig. 2 the magnetic sensor device 20 comprises eight structures 24, aligned with respect to each other. It has to be understood that this is only by way of an example and is not intended to limit the invention in any way. The magnetic sensor device 20 according to embodiments of the invention may comprise any number of structures 24 suitable or required for a particular application. Furthermore, the structures 24 may be positioned in a line, in a matrix of aligned or staggered lines, or in any other suitable regular or irregular pattern.

In, for example, biosensing processes using a magnetic sensor device 20, magnetic particles or beads are directly or indirectly attached to target moieties such as e.g. proteins, antibodies, nucleic acids (e.g. DNR, RNA), peptides, oligo- or polysaccharides or sugars, small molecules, hormones, drugs, metabolites, cells or cell fractions, tissue fractions. These molecules are to be detected in a fluid, which can be the original sample or can already be processed before insertion into the sensor device 20 (e.g. degraded, biochemically modified, filtered, or dissolved into a buffer). The original fluids can be for example, biological fluids, such as saliva, sputum, blood, blood plasma, interstitial fluid or urine, or other fluids such as drinking fluids, environmental fluids, or a fluid that results from sample pre-treatment. The fluid can for example comprise elements of solid sample material, e.g. from biopsies, stool, food, feed, environmental samples. The target moieties with attached thereto a magnetic particle will further be referred to as labeled target moieties.

The structures 24 provided in devices in accordance with embodiments of the present invention comprise a binding surface formed by the sensor surface area 23 between the structures 24 and at least part of the surface of the structures 24, for example by a top surface 25a, and at least one side surface of the structures 24, e.g. a first side surface 25b, a second side surface 25c, a third and a fourth side surface (not illustrated) in case the structure 24 is substantially rectangular, e.g. square, in cross-section, or one single side surface all around the structure in case the structure 24 is substantially circular in cross-section. Labeled target moieties can bind to the binding surface of the structures 24, for example to the top surface 25a and to at least one side surface, e.g. the first side surface 25b and the second side surface 25c, of the structures 24. This increases the total binding area of the sensor surface 23 and thus increases the sensitivity of the sensor device 20. According to embodiments of the invention, the binding surface of the structures 24 may be modified by a coating which is designed to attract certain molecules or may be modified by attaching molecules to it which are suitable to bind the target moieties which are possibly present in the sample fluid to be tested. In this way the surface of the structures 24, or at least part thereof, is activated with such binding molecules to form specific binding sites 26 to enable immobilization of the labeled target moieties to the structures 24 (see Fig. 3). In the embodiment illustrated in Fig. 3, the height h of the structures 24 may be lower than the distance from the sensor surface 23 corresponding to the cross-over point (see further) and according to this example, only the top surface may be activated with binding molecules. It has to be understood that according to other embodiments, the top surface 25a and at least one of the side surfaces, for example the first side surface 25b and the second side surface 25c, may be activated with binding molecules. According to still other embodiments of the invention, where the height of the structures 24 may be equal to the distance from the sensor surface 23 corresponding to the cross-over point (see further), target moieties binding to the top surface 25a of the structures 24 will not contribute to the sensor signal (see further). Therefore, according to these embodiments, preferably at least one of the side surfaces, for example the fist side surface 25b and the second side surface 25c, may be activated with binding molecules. Such binding molecules are know to the skilled person and may include proteins, antibodies, nucleic acids (e.g. DNA, RNA), peptides, oligo- or polysaccharides or sugars, small molecules, hormones, drugs, metabolites, cells or cell fractions, tissue fractions, .... Such molecules may be attached to the binding surface 26 of the structures 24 by means of spacer or linker molecules. Binding sites 26 on the binding surface of the structures 24 can also be provided with molecules in the form of organisms (e.g. viruses or cells) or fractions of organisms (e.g. tissue fractions, cell fractions, membranes). Specific binding sites may be formed on at least one of the top surface 25a or the side surface, e.g. the first side surface 25b or the second side surface 25c, of the structures 24. Most preferably, when the height of the structures 24 is lower than the distance from the sensor surface 23 corresponding to the cross-over point, specific binding sites 26 may be provided at all of the top surface 25a and the side surfaces of the structures 24.

According to embodiments of the invention, the height h of the structures 24 is such that labeled target moieties bound to the binding surface of the structures 24 positively contribute to a sensor signal, the sensor signal being representative for the presence and/or amount of labeled target moieties bound to the binding surface of the structures 24.

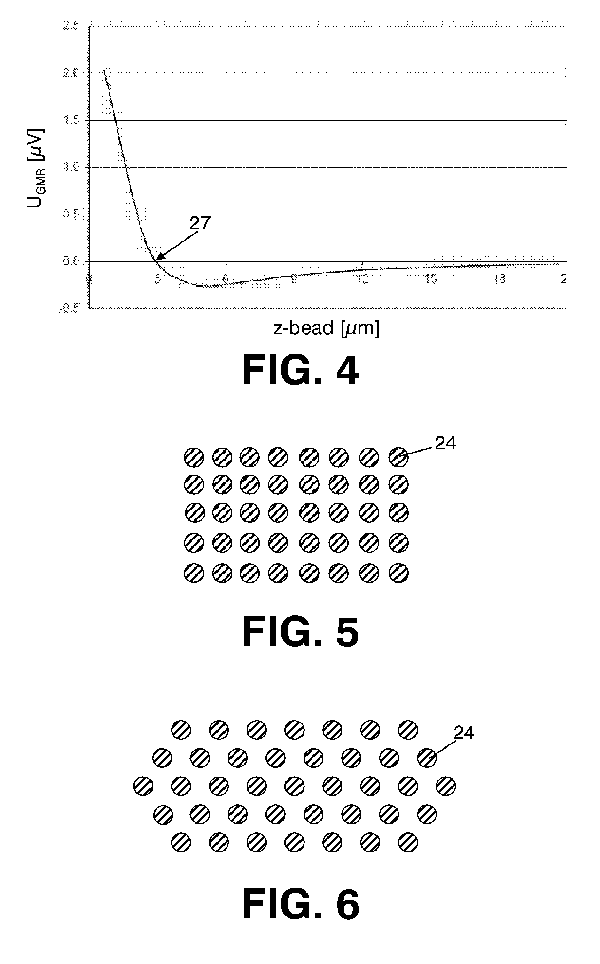

Fig. 4 shows the signal distribution of a filled plane of magnetic particles to a sensor signal as a function of the distance z of the top surface of the filled plane above the sensor surface 23 in the z-direction (see Fig. 2) for a GMR element 21 in a configuration as illustrated in Figs. 2 and 3, i.e. a GMR element 21 sandwiched in between two current wires 22. With a filled plane of magnetic particles at a distance z above the sensor surface is meant that there is a homogenous bead distribution in the space between the sensor surface 23 and the distance z above the sensor surface. It can be seen from Fig. 4 that the contribution of the homogeneously distributed magnetic particles to the sensor signal is rapidly decreasing with increasing distance z of the filled plane of magnetic particles from the sensor surface 23 in the z-direction. In other words, the sensitivity of the GMR element 21 with respect to a filled plane of magnetic particles decreases with increasing distance z of the filled plane of magnetic particles above the sensor surface 23, up to a point where the contribution of the magnetic particles to the sensor signal becomes negative when these magnetic particles are too far away from the sensor surface 23 and thus from the GMR element 21.

Negative contribution of the magnetic particles to the sensor signal can be explained as follows. In the configuration as illustrated in Figs. 2 and 3, current is sent through both the first and second current wire 22 for respectively generating a first and second magnetic field. Both the first and second magnetic fields have a magnetizing effect on the magnetic particles present at a certain distance above the sensor surface 23. Because the magnetic particles have a north pole and a south pole, the result of both the first and second magnetic field influencing the magnetic particles, and the influence from the magnetic dipoles on each other, is that immediately above the sensor surface the magnetic particles have a positive contribution to the sensor signal or, in other words, help to increase the sensor signal, while from a certain distance above the sensor surface on (illustrated as 3 µm in Fig. 4), the magnetic particles have a negative contribution to the sensor signal, or in other words, decrease the sensor signal.

In the example given in Fig. 4 it can be seen that for a distance z smaller than 3 µm the signal contribution of a filled plane of magnetic particles to the sensor signal is positive, while for a distance larger than 3 µm the signal contribution of the filled plane of magnetic particles to the sensor signal is negative. Hence, a homogenous distribution of magnetic particles in a space above the sensor surface 23, in the example illustrated above 3 µm, will cause a partial cancelling of the sensor signal because the integral of the negative part has to be subtracted from the integral of the positive part. The integral of the negative part may even be larger than the integral of the positive part for the present configuration of the magnetic sensor device 20. Arrow 27 indicates the point of change between the positive and negative signal contribution of magnetic particles to the sensor signal, or in other words, the point or distance from the sensor surface 23 where the signal contribution of the magnetic particles to the sensor signal is equal to zero. In the following description, this point will be referred to as signal cross-over point 27.

The signal cross-over point 27 may be dependent on the kind of magnetic elements 21 used and on the configuration of magnetic elements 21 and magnetic field generating means 22 used to form the magnetic sensor device 20. The signal cross-over point 27 can be determined for any configuration before forming the at least one structure 24 on the sensor surface 23.

For example, when only one current wire 22 is used for forming the magnetic field generator the main difference with the configuration where two current wires 22 are used is the signal strength obtained. With two current wires 22 the signal strength is substantially higher. The main reason for the behavior of the curves is that the magnetic field lines around the current wires 22 have a circular shape according to Biot-Savart's law. Depending on the position of a magnetic particle within this field with respect to the current wire the magnetic particle is magnetized in a different direction. The magnetized magnetic particle forms an approximated dipole stray field that is picked up by the sensor element. Depending on the position of the magnetized magnetic particle with respect to the sensor element the orientation and strength of the dipole field is different. By assuming a magnetic particle distribution in space and integrating the contributions from all magnetic particles a curves similar to the one illustrated in Fig. 4 for the use of two current wires 22 may be obtained, except that the maximal signal obtained now is about 1 µV instead of 2 µV in case of Fig. 4.

Because of the above, according to embodiments of the present invention, the height h of the structures 24 should be higher than zero and lower than or equal to a distance z of the labeled target moieties from the sensor surface 23 corresponding to the signal cross-over point 27 from positive to negative signal contribution. This distance z where the signal cross-over point is reached may be different for different sensor configurations. By keeping the height h of the structures 24 lower than the distance z of the cross-over point 27, the labeled target moieties bound to the binding surface 25 of at least one structure 24 will only positively contribute to the sensor signal. With the labeled target moieties positively contributing to the sensor signal is meant that the presence of all or substantially all of the labeled target moieties bound to the binding surface 25 of the at least one structure does not cancel or does not substantially cancel the signal. Hence, all, or substantially all, labeled target moieties which are bound to the binding surface 25 of the at least one structure 24 contribute to an increase of the measured sensor signal, which is representative for the presence and/or amount of labeled target moieties bound to the binding surface 25. In this way, the amount of bound labeled target moieties to the binding surface 25 of the at least one structure 24 can be correctly derived from the measured sensor signal, as all bound labeled target moieties lead to an increase of the sensor signal. This increases the sensitivity of the magnetic sensor device 20.

Because all or substantially all labeled target moieties which are bound to the binding surface 25 of the at least one structure 24 of the magnetic sensor device 20 positively contribute to the sensor signal and thus no cancelling of the sensor signal occurs, the magnetic sensor device 20 according to embodiments of the present invention provides good signals and thus show good sensitivity.

According to a preferred embodiment of the invention the height h of the structures 24 may be equal to a distance z of the labeled target moieties from the sensor surface 23, the distance z corresponding to the signal cross-over point from positive to negative signal contribution of the labeled target moieties which are bound to the binding surface 25 of the structures 24. An advantage of this embodiment is that the contribution of the bound labeled target moieties to the sensor signal is maximal and thus the sensor signal is maximal. A magnetic sensor device 20 comprising structures 24 having a height h equal to a distance z of the labeled target moieties from the sensor surface 23 corresponding to the signal cross-over point 27 shows a good sensitivity.

The average signal from one magnetic particle on the side of a cylinder, the cylinder having a height equal to the distance of the labeled target moieties from the sensor surface corresponding to a signal cross-over point from positive to negative signal contribution, for the example illustrated in Fig. 4 a 3 µm high cylinder, will be about 1/3 of the signal resulting from a magnetic particle on the sensor surface 23. Therefore, the increase in surface by applying structures 24 extending from the sensor surface 23, e.g. pillars, will only have an effect if the obtained increase in binding surface area is more than 3 times the surface area of the structure 24 on the sensor surface 23. For example, for a circular structure 24 with a radius of r µm and a height equal to the distance of the labeled target moieties from the sensor surface corresponding to a signal cross-over point from positive to negative signal contribution, e.g. 3 µm, a gain in surface area, with respect to a sensor surface 23 having no such structures 24, determined by the surface of the cylinder (top not included) (which surface is determined by 2πrh) decreased with the surface area determined by those parts of the sensor surface 23 which are covered by the structures 24 (determined by πr²) may be obtained. Hence, for the example of a 3 µm high structure, a 2πrh-πr² = 6πr-πr² times larger surface area and consequently, on average, a (6πr-πr²)/3 times larger signal may be obtained.

The distance d between neighboring structures 24 may preferably be such that a capillary flow of sample fluid over the sensor surface 23 is obtained. Preferably, the distance d between neighboring structures 24 may be between 1 µm and 100 µm and most preferably between 1 µm and 70 µm. The structures 24 may preferably have a width w of between 0.5 µm and 10 µm. The lower limit of the width w may be limited by the technique used to form the structures 24. With current techniques, such as e.g. soft lithography or hot embossing, structures 24 with a width w of 0.5 µm can be obtained.

The structures 24 may be placed on the sensor surface 23 in an array comprising rows and columns of structures 24, as illustrated in Fig. 5 which shows a top view of a magnetic sensor device 20 according to an embodiment of the invention. According to other embodiments of the present invention, the structures 24 may be placed on the sensor surface 23 in an array of staggered rows and/or columns, as illustrated in Fig. 6 which shows a top view of a magnetic sensor device 20 according to an embodiment of the present invention. According to yet other embodiments of the present invention, the structures 24 may be randomly be placed on the sensor surface 23 in regular or irregular patterns (not illustrated).

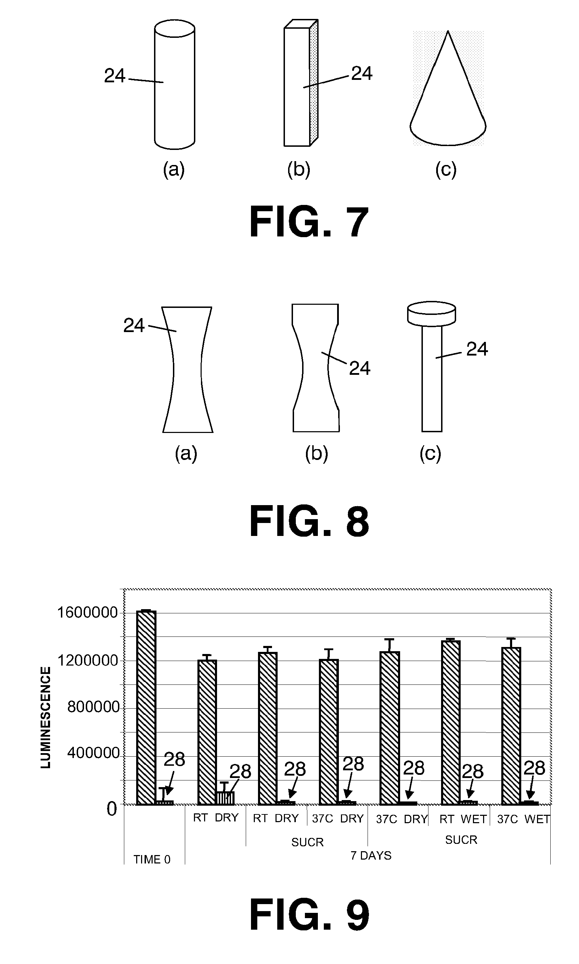

In the above embodiments illustrated in Fig. 5 and Fig. 6, the structures 24 are illustrated as having the shape of a cylinder. However, other than a cylindrical shape (see Fig. 7(a)), the structures 24 may also have the shape of a beam (see Fig. 7(b)) or of a cone (see Fig. 7(c)). The shapes illustrated in Fig. 7(a) and Fig. 7(b) have in all cross-sections parallel to the sensor surface 23 the same shape and dimensions. According to still other embodiments of the present invention, the structures 24 may have shapes as illustrated in Fig. 8(a) to 8(c), which in different cross-sections parallel to the sensor surface 23 may have different shapes and/or dimensions. The structures 24 may have any other suitable shape. The structures illustrated in Fig. 7 are easier to make than the structures illustrated in Fig. 8, as they can for example be released from a mould or stamp.

The presence of at least one structure 24 on the sensor surface 23 enhances the surface-to-volume ratio of the sensor surface 23 and will maintain a 'creep flow' over the sensor surface 23 by capillary actuation of the fluid. Furthermore, due to the structures 24 as described above, realized by e.g. imprinting or lithographic techniques, the variation that is present in porous material may be eliminated.

In addition, the presence of the structures 24 on the sensor surface 23 allows easy magnetic actuation of labeled target moieties from and to the binding surface 25a, 25b, 25c of the structures 24, thereby enhancing the efficiency of the binding process and subsequent bound/free process (= separation bound and non-bound magnetic particles). Magnetic actuation of the labeled target moieties may be done by using top and bottom magnets, i.e. a magnet located under a substrate on which the magnetic sensor device is formed and one above the structures 24 present at the sensor surface 23, for transporting the labeled target moieties perpendicularly to the sensor surface 23. The top and bottom magnets may, for example, be formed by coils or by moveable permanent magnets.

In a second aspect of the present invention, a method is provided for manufacturing a magnetic sensor device 20 in accordance with embodiments of the present invention. The method comprises providing at least one structure 24 on a sensor surface 23 lying in a plane, the at least one structure 24 having a height h and a binding surface 25a, 25b, 25c for binding labeled target moieties and extending from the sensor surface 23 in a direction substantially perpendicular to the plane of the sensor surface 23 over the height h. The method furthermore comprises providing at least one magnetic sensor element 21, e.g. GMR element or any other suitable magnetic detection element, for providing a sensor signal representative for the presence and/or amount of labeled target moieties bound to the binding surface 25a, 25b, 25c of the at least one structure 24. According to embodiments of the present invention, providing at least one structure 24 is performed such that the height h of the at least one structure 24 is higher than zero and lower or equal to a distance z of the labeled target moieties from the sensor surface 23 which corresponds to a signal cross-over point 27 from positive to negative signal contribution of the labeled target moieties to the sensor signal, the signal cross-over point 27 being the distance where the signal contribution of the bound labeled target moieties to the sensor signal is equal to zero.

According to embodiments of the invention, providing the at least one structure 24 may be performed such that the height h of the at least one structure is such that the labeled target moieties bound to the binding surface 25a, 25b, 25c of the at least one structure 24 positively contribute to the sensor signal. With the labeled target moieties positively contributing to the sensor signal is meant that the presence of all or substantially all of the labeled target moieties bound to the binding surface 25a, 25b, 25c of the at least one structure 24 does not or not substantially cancel the signal. Thus all or substantially all labeled target moieties bound to the binding surface 25a, 25b, 25c of the at least one structure 24 contribute to an increase of the measured sensor signal, which is representative for the presence and/or the amount of bound labeled target moieties. In this way, the amount of labeled target moieties which are bound to the binding surface 25a, 25b, 25c of the at least one structure 24 can be correctly derived from the measured sensor signal, as all bound labeled target moieties lead to an increase of the sensor signal.

The method according to embodiments of the invention will be described using the configuration of the sensor device 20 illustrated in Figs. 2 and 3. It has to be understood that this is only by way of an example and that other configurations of magnetic sensor devices 20 can also be formed using the method according to embodiments of the present invention.

Providing the structures 24 on the sensor surface 23 may be performed by providing a layer of a polymer material or a layer of an inorganic material such as Si₃N₄, SiO₂ or any other suitable inorganic material which is commonly used in e.g. integrated circuit fabrication, onto the sensor surface 23 and patterning the layer of polymer or inorganic material. When the magnetic sensor device 20 is a biosensor, the polymer or inorganic material used to provide the layer from which the structures 24 will be formed may most preferably chosen such that it is biocompatible. Most preferably, this layer may be formed from a polymer. The polymer used to provide the polymer layer may be any suitable polymer material known by a person skilled in the art and suitable for forming structures 24. Patterning the layer of polymer or inorganic material may be performed by, for example, imprinting techniques such as hot-embossing or by lithographic techniques such as by photolithographic patterning.

According to preferred embodiments, providing the structures 24 on the sensor surface 23 may be performed such that the height h of the structures 24 is equal to a distance z of the labeled target moieties from the sensor surface 23 corresponding to the signal cross-over point 27 from positive to negative signal contribution of the labeled target moieties to the sensor signal. An advantage of these embodiments is that the contribution of the bound labeled target moieties to the sensor signal is maximal and thus the sensor signal is maximal. A magnetic sensor device 20 comprising structures 24 having a height equal to a distance z of the labeled target moieties from the sensor surface 23 corresponding to the signal cross-over point 27 shows a good sensitivity.

Providing the structures 24 may, according to embodiments of the invention, be performed such that they are arranged over the sensor surface 23 in a matrix, the matrix comprising rows and columns. The matrix may be a regular or a staggered matrix. According to other embodiments of the invention, providing the structures 24 may be performed such that they are arranged over the sensor surface 23 in regular or irregular patterns. According to embodiments of the invention, providing the structures 24 may be performed such that they are randomly arranged over the sensor surface 23.

The method according to embodiments of the invention may furthermore comprise providing a magnetic field generating means for applying a magnetic field to the magnetic particles. According to embodiments of the invention, the magnetic field generating means may be an on-chip magnetic field generating means, such as current wires 22, or may be an off-chip magnetic field generating means, such as an external permanent magnet.

According to preferred embodiments of the present invention, the structures 24 may be formed by patterning an epoxy-based SU-8 polymer layer. Therefore, a layer of SU-8 polymer is provided on the sensor surface 23 of the sensor device 20. This can be done by any suitable deposition technique known by a person skilled in the art. Then, according to the example given, photolithographic patterning may be performed for forming the structures 24. The photolithography process may comprise the following subsequent steps. First, a photoresist layer is applied on top of the SU-8 polymer layer, for example by means of spincoating. The photoresist layer may for example have a thickness of a few µm and may be made of any suitable polymer that can be used as a photoresist, such as for example poly(vinyl cinnamate) or novolak-based polymers. Thereafter, a mask is applied to align a pattern onto the SU-8 polymer layer. The photoresist layer is then illuminated through the mask e.g. by means of UV light. After illumination the photoresist is developed by which either the illuminated parts of the photoresist (positive resist) or the non-illuminated parts of the photoresist (negative resist) are removed, depending on which type of photoresist has been used. Patterning of the SU-8 polymer layer is then performed using the developed photoresist layer as a mask, after which the remaining parts of the photoresist layer are removed, typically by using an organic solvent. In this way, a sensor surface 23 may be obtained comprising at least one structure 24 extending from the sensor surface 23 in a direction substantially perpendicular to the plane of the sensor surface 23 over a height h, the structures 24 being formed of SU-8 polymer material.

Fig. 9 illustrates that on SU-8 surfaces the binding efficiency of binding molecules, such as antigens which in the example given may be BSA-morphin (see bars filled with slanted lines in Fig. 9), which form binding sites 26, is high. This may be due to covalent coupling of the BSA-morphin to epoxy groups of the epoxy-based polymer SU-8. In Fig. 9 results are shown of an assay performed on SU-8 coated disks. The SU-8 coated surface is covered with BSA-Morphin antigens to which target moieties, in the example given antibodies, can bind once they reach the surface. As a control, SU-8 surfaces were covered with only BSA protein (see little bars indicated with reference number 28 in Fig. 9), without the antigen. The antibodies that remain on the surface after washing were labeled with a horseradish peroxidase (HRP)-tagged secondary antibody. HRP is an enzyme that catalyses conversion of luminol, which releases photons. Luminescence may then be measured upon incubation with luminol, which is a measure for the amount of beads on the surface. Assays were performed after 0 and 7 days under different storage conditions i.e. at room temperature or 37°C, dry or wet and with or without a protective sucrose/BSA coating. The sucrose/BSA coating may function as a protection layer for protecting the BSA proteins and to improve wetting of the surface. Although there is some loss in signal in time, even under harsh conditions (37 °C) the SU-8 surfaces still perform well after 7 days.

From the above results it is clear that the epoxy-base SU-8 polymer is a good candidate to be used for forming the structures 24 on the sensor surface 23 of the magnetic sensor device 20 according to embodiments of the present invention, especially when the magnetic sensor device is to be used for molecular diagnostics or biological sample analysis.

The present invention also provides, in a third aspect, a method for determining the presence and/or amount of target moieties in a sample fluid. The method comprises providing a sample fluid comprising labeled target moieties to a magnetic sensor device 20 as described in embodiments according to the present invention. The method according to embodiments of the third aspect of the present invention then comprises applying a magnetic field for magnetizing the magnetic or magnetizable objects, e.g. magnetic particles, attached to the target moieties which are bound to the binding surface 25a, 25b, 25c of the at least one structure 24 on the sensor surface 23, measuring the sensor signal by means of the at least one magnetic element, e.g. GMR element 21, and from the measured sensor signal determining the presence and/or amount of target moieties bound to the binding surface 25 of the at least one structure 24.

According to embodiments of the present invention, the sample fluid may comprise labeled target moieties before it is applied to the sensor device 20. According to other embodiments of the invention, providing a sample fluid comprising labeled target moieties to the magnetic sensor device 20 may be performed by applying a sample fluid comprising target moieties to the magnetic sensor device followed by adding magnetic or magnetizable particles to the sample fluid. According to yet other embodiments of the invention, providing a sample fluid comprising labeled target moieties to the magnetic sensor device 20 may be performed by first adding magnetic or magnetizable particles to the sample fluid and then applying the sample fluid comprising the labeled target moieties to the magnetic sensor device 20.

The method according to this aspect of the present invention may be used in molecular diagnostics, biological sample analysis or chemical sample analysis.

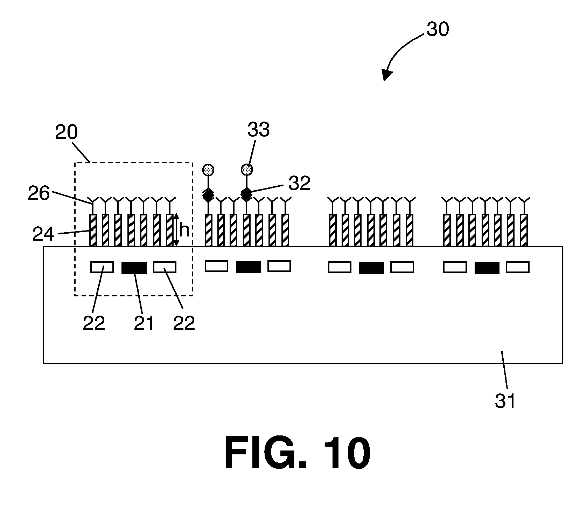

In a further aspect, the present invention also provides a biochip 30 comprising at least one magnetic sensor device 20 according to embodiments of the present invention. Fig. 10 illustrates a biochip 30 according to an embodiment of the present invention. The biochip 30 may comprise at least one magnetic sensor device 20 according to embodiments of the present invention integrated in a substrate 31. The term "substrate" may include any underlying material or materials that may be used, or upon which a device, a circuit or an epitaxial layer may be formed. The term "substrate" may include a semiconductor substrate such as e.g. a doped silicon, a gallium arsenide (GaAs), a gallium arsenide phosphide (GaAsP), an indium phosphide (InP), a germanium (Ge), or a silicon germanium (SiGe) substrate. The "substrate" may include, for example, an insulating layer such as a SiO₂ or a Si₃N₄ layer in addition to a semiconductor substrate portion. Thus the term "substrate" also includes glass, plastic, ceramic, silicon-on-glass, silicon-on-sapphire substrates. The term "substrate" is thus used to define generally the elements for layers that underlie a layer or portions of interest. Also the "substrate" may be any other base on which a layer is formed, for example a glass or metal layer.

According to embodiments of the invention a single magnetic sensor device 20 or a multiple of magnetic sensor devices 20 may be integrated on the same substrate 31 to form the biochip 30.

According to the present example, the magnetic sensor devices 20 integrated in the substrate 31 of the biochip 30 comprise a sensor surface 23 lying in a plane and provided with at least one structure 24 extending from the sensor surface 23 in a direction substantially perpendicular to the plane of the sensor surface 23 over a height h, the at least one structure 24 having a binding surface for binding target moieties 32 labeled with magnetic or magnetizable objects 33, e.g. magnetic particles, and at least one magnetic sensor element 21 for providing a sensor signal representative for the presence and/or amount of labeled target moieties bound to the binding surface 25 of the at least one structure 24. According to the present invention, the height h of the at least one structure 24 is higher than zero and lower or equal to a distance z of the labeled target moieties from the sensor surface 23 corresponding to a signal cross-over point 27 from positive to negative signal contribution of the labeled target moieties 32 to the sensor signal, the signal cross-over point 27 being the distance where the signal contribution of the bound labeled target moieties 32 to the sensor signal is equal to zero.

In each magnetic sensor device 20 at least one sensor element 21, for example a GMR element, may be integrated in the substrate 31 to read out the information gathered by the biochip 30, thus for example to read out the presence or absence of target moieties 32 via magnetic or magnetizable objects 33, e.g. magnetic particles, attached to the target moieties 32, thereby determining or estimating an areal density of the target particles 33. The magnetic or magnetizable objects 33, e.g. magnetic particles, are preferably implemented by so called superparamagnetic beads. Binding sites 26 which are able to selectively bind a target moiety 32 are attached to the binding surface 25 of the at least one structure 24.

Each of the magnetic sensor devices 20 in the biochip 30 may furthermore comprise a magnetic field generating means, in the example given formed by two current wires 22. According to embodiments of the invention, the magnetic field generating means may be an on-chip magnetic field generating means, such as the current wires 22 in the example given, or may be an off-chip magnetic field generating means, such as an external permanent magnet.

The functioning of the biochip 30, and thus also of the magnetic sensor device 20, will be explained hereinafter. Each binding site 26 may be of a certain type, for binding pre-determined target moieties 32. A target sample, comprising target moieties 32 to be detected, may be presented to or passed over the binding sites 26 of the biochip 30, and if the binding sites 26 and the target moieties 32 match, they bind to each other. The superparamagnetic beads 33, or more generally the magnetic or magnetizable objects, may be directly or indirectly coupled to the target moieties 32. The magnetic or magnetizable objects, e.g. superparamagnetic beads 33, allow reading out the information gathered by the biochip 30.

In addition to molecular assays, also larger moieties can be detected, e.g. cells, viruses, or fractions of cells or viruses, tissue extract, etc. Detection can occur with or without scanning of the sensor element 21 with respect to the biosensor surface 23.

Measurement data can be derived as an end-point measurement, as well as by recording signals kinetically or intermittently.

The magnetic or magnetizable objects 33, e.g. magnetic particles, can be detected directly by the sensing method. As well, the magnetic or magnetizable objects 33, e.g. magnetic particles, can be further processed prior to detection. An example of further processing is that materials are added or that the (bio)chemical or physical properties of the magnetic or magnetizable objects 33, e.g. magnetic particles, are modified to facilitate detection.

The magnetic sensor device 20 and biochip 30 according to embodiments of the present invention can be used with several biochemical assay types, e.g. binding/unbinding assay, sandwich assay, competition assay, displacement assay, enzymatic assay, etc.

The magnetic sensor device 20 and biochip 30 according to embodiments of this invention are suitable for sensor multiplexing (i.e. the parallel use of different sensors and sensor surfaces), label multiplexing (i.e. the parallel use of different types of labels or magnetic or magnetizable objects 33) and chamber multiplexing (i.e. the parallel use of different reaction chambers).

The magnetic sensor device 20 and biochip 30 according to embodiments of the present invention can be used as rapid, robust, and easy to use point-of-care biosensors for small sample volumes. The reaction chamber can be a disposable item to be used with a compact reader, containing the one or more magnetic field generating means and one or more detection means. Also, the device 20 and biochip 30 according to the present invention can be used in automated high-throughput testing. In this case, the reaction chamber may, for example, be a well plate or cuvette, fitting into an automated instrument.

It is to be understood that although preferred embodiments, specific constructions and configurations, as well as materials, have been discussed herein for devices according to the present invention, various changes or modifications in form and detail may be made without departing from the scope and spirit of this invention.

## Claims

1. Magnetic sensor device (20) for determining the presence and/or amount of target moieties (32) in a sample fluid, the target moieties (32) being labeled with magnetic or magnetizable objects (33), the magnetic sensor device (20) comprising:
- a sensor surface (23) lying in a plane,
- at least one structure (24) extending from the sensor surface (23) in a direction substantially perpendicular to the plane of the sensor surface (23), the at least one structure (24) having a height (h) and a binding surface (25a, 25b, 25c) for binding labeled target moieties, and
- at least one magnetic sensor element (21) for providing a sensor signal representative for the presence and/or amount of labeled target moieties (32) bound to the binding surface (25a, 25b, 25c) of the at least one structure (24),
wherein the height (h) of the at least one structure (24) is higher than zero and lower than or equal to a distance (z) of the labeled target moieties (32) from the sensor surface (23) corresponding to a signal cross-over point (27) from positive to negative signal contribution of the labeled target moieties (32) to the sensor signal, the signal cross-over point (27) being the distance at which a homogeneous distribution of magnetic or magnetizable objects (33) between the sensor surface (23) and that distance has a signal contribution to the sensor signal equal to zero.

2. Magnetic sensor device (20) according to claim 1, wherein the height (h) of the at least one structure (24) is equal to the distance (z) of the labeled target moieties (32) from the sensor surface (23) corresponding to the signal cross-over point (27) from positive to negative signal contribution of the labeled target moieties (32) at a distance from the sensor surface (23) to the sensor signal.

3. Magnetic sensor device (20) according to claim 1 or 2, the at least one structure (24) having a width (w), wherein the width (w) of the at least one structure (24) is between 0.5 µm and 10 µm.

4. Magnetic sensor device (20) according to any of the previous claims, the magnetic sensor device (20) comprising a plurality of structures (24) and there being a distance (d) between neighboring structures (24), wherein the distance (d) between neighboring structures (24) is between 1 µm and 70 µm.

5. Magnetic sensor device (20) according to any of the previous claims, the magnetic sensor device (20) comprising a plurality of structures (24), wherein the plurality of structures (24) is arranged over the sensor surface (23) in a matrix, the matrix comprising rows and columns.

6. Magnetic sensor device (20) according to any of the previous claims, furthermore comprising magnetic field generating means (22).

7. Magnetic sensor device (20) according to any of the previous claims, the at least one structure (24) having an upper surface (25a) and at least one side surface (25a, 25b), wherein the binding surface (25) of the at least one structure (24) is formed by its upper surface (25a), and/or its at least one side surface (25b, 25c).

8. Magnetic sensor device (20) according to claim 7, wherein at least one of the upper surface (25a) and the at least one side surface (25b, 25c) of the at least one structure (24) comprises specific binding sites (26).

9. Magnetic sensor device (20) according to any of the previous claims, wherein the at least one structure (24) is formed of a polymer or inorganic material.

10. Magnetic sensor device (20) according to claim 9, wherein the at least one structure (24) is formed of an SU-8 polymer.

11. Magnetic sensor device (20) according to any of the previous claims, wherein the at least one structure (24) has a rectangular or cylindrical shape in cross-section.

12. Magnetic sensor device (20) according to any of the previous claims, wherein the magnetic or magnetizable objects (33) are magnetic particles.

13. Use of the magnetic sensor device (20) according to any of claims 1 to 12 in molecular diagnostics, biological sample analysis or chemical sample analysis.

14. A biochip comprising at least one magnetic sensor device (20) according to any of claims 1 to 12.

15. Use of the biochip according to claim 14 in molecular diagnostics, biological sample analysis or chemical sample analysis.

16. Method for manufacturing a magnetic sensor device (20), the method comprising:
- providing at least one structure (24) on a sensor surface (23) lying in a plane, the at least one structure (24) extending from the sensor surface (23) in a direction substantially perpendicular to the plane of the sensor surface (23) over a height (h) and having a binding surface (25) for binding target moieties (32) labeled with magnetic or magnetizable objects (33), and
- providing at least one magnetic sensor element (21) for providing a sensor signal representative for the presence and/or amount of labeled target moieties (32) bound to the binding surface (25) of the at least one structure (24),
wherein providing at least one structure (24) is performed such that the height (h) of the at least one structure (24) is higher than zero and lower than or equal to a distance (z) of the labeled target moieties (32) from the sensor surface (23) corresponding to a signal cross-over point (27) from positive to negative signal contribution of the labeled target moieties (32), the signal cross-over point (27) being the distance at which a homogeneous distribution of magnetic or magnetizable objects (33) between the sensor surface (23) and that distance has a signal contribution to the sensor signal equal to zero.

17. Method according to claim 16, wherein providing at least one structure (24) is performed such that the height (h) of the at least one structure (24) is equal to the distance (z) of the labeled target moieties (32) from the sensor surface (23) corresponding to the signal cross-over point (27) from positive to negative signal contribution of the labeled target moieties (32) to the sensor signal.

18. Method according to claim 16 or 17, wherein a plurality of structures (24) is provided, the plurality of structures (24) being arranged over the sensor surface (23) in a matrix, the matrix comprising rows and columns.

19. Method for determining the presence and/or amount of target moieties (32) in a sample fluid, the method comprising:
- providing a sample fluid comprising labeled target moieties (32) to a magnetic sensor device (20) comprising a sensor surface (23) lying in a plane, at least one magnetic element (21) for providing a sensor signal, and at least one structure (24) having a height (h) higher than zero and lower or equal to a distance (z) of the labeled target moieties (32) from the sensor surface (23) corresponding to a signal cross-over point (27) from positive to negative signal contribution of the labeled target moieties (32) to the sensor signal, the signal cross-over point (27) being the distance at which a homogeneous distribution of magnetic or magnetizable objects (33) between the sensor surface (23) and that distance has a signal contribution to the sensor signal equal to zero,
- applying a magnetic field for magnetizing the magnetic or magnetizable objects (33) bound to target moieties (32) bound to the binding surface (25) of the at least one structure (24),
- measuring the sensor signal by means of the magnetic element (21), and
- from the measured sensor signal determining the presence and/or amount of target moieties (32) bound to the binding surface (25) of the at least one structure (24).

20. Method according to claim 19, wherein providing the sample fluid comprising labeled target moieties (32) to the sensor device (20) comprises:
- first applying a sample fluid comprising target moieties (32) to the magnetic sensor device (20), and
- thereafter adding magnetic or magnetizable objects (33) to the sample fluid.

21. Method according to claim 19, wherein providing the sample fluid comprising labeled target moieties (32) to the sensor device (20) comprises:
- first adding magnetic or magnetizable objects (33) to a sample fluid comprising target moieties, and
- thereafter applying the sample fluid to the magnetic sensor device (20).

22. Use of the method according to any of claims 19 to 21 in molecular diagnostics, biological sample analysis or chemical sample analysis.
